# EUROPÄISCHE PATENTSCHRIFT

(11) **EP 0 519 327 B1**
(45) Veröffentlichungstag und Bekanntmachung des Hinweises auf die Patenterteilung: **08.09.1999**
(21) Anmeldenummer: 92109834.9
(22) Anmeldetag: 11.06.1992
(51) Int. Cl.: C07C 323/59, A61K 38/00

(54) **N-Acyl-S-(2-hydroxyalkyl)-cysteine, deren Herstellung sowie deren Verwendung als Zwischenprodukte zur Herstellung von synthetischen Immunadjuvantien und synthetischen Impfstoffen**
N-Acyl-S-(2-hydroxyalkyl)-cysteines, their preparation and their use as intermediates in the preparation of synthetic immunoadjuvants and synthetic vaccines
N-Acyl-S-(2-hydroxyalkyl)-cystéines, leur préparation et leur emploi comme intermédiaires dans la préparation de immuno-adjuvants synthétiques et vaccins synthétiques

(30) Priorität: 17.06.1991 DE 4119856
(43) Veröffentlichungstag der Anmeldung: 23.12.1992
(73) Patentinhaber: HOECHST AKTIENGESELLSCHAFT, 65926 Frankfurt am Main (DE)
(72) Erfinder: Metzger, Jörg, Dr., W-7400 Tübingen (DE); Wiesmüller, Karl-Heinz, Dr., W-7400 Tübingen (DE); Jung, Günther, Dr. Prof Dr., W-7400 Tübingen (DE)

(56) Entgegenhaltungen:
- EP-A- 0 000 330
- EP-A- 0 014 815
- EP-A- 0 114 787
- LIEBIGS ANNALEN DER CHEMIE. Bd. 1983, Nr. 9, 1983, WEINHEIM DE Seiten 1608 - 1622; G. JUNG ET AL: 'THE MITOGENIC PRINCIPLE OF ESCHERICHIA COLI LIPOPROTEIN'
- INTERNATIONAL JOURNAL OF PEPTIDE AND PROTEIN RESEARCH Bd. 38, Nr. 6, Dezember 1991, COPENHAGEN DK Seiten 545 - 554; J. W. METZGER ET AL: 'SYNTHESIS OF N-FMOC PROTECTED DERIVATIVES OF S-(2,3-DIHYDROXYPROPYL)-CYSTEINE AND THEIR APPLICATION IN PEPTIDE SYNTHESIS'
- CHEMICAL AND PHARMACEUTICAL BULLETIN. Bd. 39, Nr. 3, März 1991, TOKYO JP Seiten 607 - 611; Y. TSUDA ET AL: 'STRUCTURE AND SYNTHESIS OF AN IMMUNOACTIVE LIPOPEPTIDE, WS1279, OF MICROBIAL ORIGIN'
- CHEMICAL AND PHARMACEUTICAL BULLETIN. Bd. 38, Nr. 4, April 1990, TOKYO JP Seiten 1110 - 1112; M. KURIMURA ET AL: 'SYNTHESIS OF BIOLOGICALLY ACTIVE PENTAPEPTIDE ANALOGS OF THE N-TERMINAL PART OF LIPOPROTEIN FROM THE OUTER MEMBRANE OF ESCHERICHIA COLI'

## Beschreibung

Die vorliegende Erfindung betrifft N-Acyl-S-(2-hydroxyalkyl)-cysteinen, ein Verfahren zu deren Herstellung und deren Verwendung als Zwischenprodukte zur Herstellung von synthetischen Immunadjuvantien und synthetischen Impfstoffen.

Einige Vertreter der genannten Verbindungsklasse sind bereits bekannt (EP 0 000 330). Mit den bekannten Verbindungen läßt sich jedoch nur eine begrenzte Anzahl an Lipopeptiden herstellen.

Überraschenderweise wurde nun gefunden, daß sich mittels neuer N-Acyl-S-(2-hydroxyalkyl)-cysteine viele Lipopeptide vorteilhaft herstellen lassen.

Erfindungsgegenstand sind demzufolge Verbindungen der Formel I 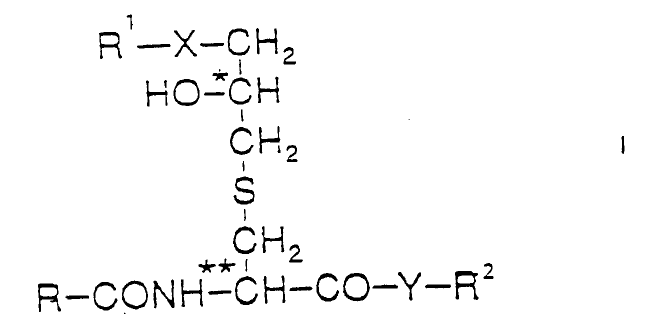 in der R einen Fluorenylmethyloxy bedeutet, X Sauerstoff (O) bedeutet, Y Sauerstoff ist, R¹ Wasserstoff, einen gesättigten aliphatischen Kohlenwasserstoffrest mit 9 bis 18 C-Atomen oder einen Rest der Formel C(O)-R³ bedeutet, wobei R³ ein gesättigter Kohlenwasserstoffrest mit 9 bis 17 C-Atomen ist, und R² tert.-Butyl, bedeutet, Die Verbindungen besitzen an den mit * bzw. ** bezeichneten Asymmetrie-Zentren die absolute R- bzw. S-Konfiguration und liegen als reine Diastereomere oder gegebenenfalls als Diastereomerengemische vor.

Bevorzugt sind Verbindungen, die an den mit * bzw. ** bezeichneten Asymmetrie-Zentren die absolute R-Konfiguration besitzen.

Die beschriebenen Verbindungen eignen sich insbesondere als Zwischenprodukte zur Herstellung von synthetischen Adjuvantien (EP 0 000 330; US-Patent 4 666 886, B-Lymphozyten und Makrophagen Stimulantien (P. Hoffmann, K.-H. Wiesmüller, J. Metzger, G. Jung, W.G. Bessler, Biol. Chem. Hoppe-Seyler 370 (1989) 575) und synthetischen Impfstoffen; Wiesmüller, K.-H., Jung, G. Hess, G. Vaccine 7 (1989) 29, DE-OS-35 46 150).

Zum Gegenstand der vorliegenden Erfindung gehört weiterhin ein Verfahren zur Herstellung von Verbindungen der Formel I', 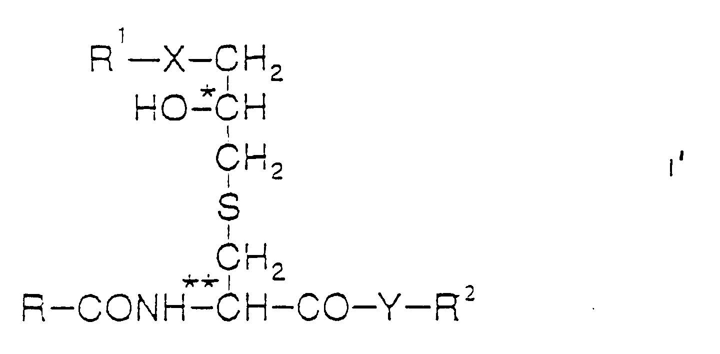 in der R einen Fluorenylmethyloxy-, tert.-Butyloxy-, Benzyloxy-Rest oder einen gesättigten oder ungesättigten, aliphatischen oder gemischt aliphatisch-cycloaliphatischen, gegebenenfalls durch Sauerstoffunktionen oder Halogenatome substituierten Kohlenwasserstoffrest mit 1 bis 21 C-Atomen bedeutet, X Sauerstoff (O) oder Methylen (CH₂) bedeutet, Y Sauerstoff oder ein gegebenenfalls mit einer in der Peptidchemie üblichen Schutzgruppen seitenkettengeschützter Aminosäurerest ist, R¹ Wasserstoff, einen gesättigten oder ungesättigten, aliphatischen oder gemischt aliphatisch-cycloaliphatischen, gegebenenfalls durch Sauerstoffunktionen oder Halogenatome substituierten Kohlenwasserstoffrest mit 1 bis 21 C-Atomen oder einen Rest der Formel C(O)-R³ bedeutet, wobei R³ ein gesättigter oder ungesättigter, aliphatischer oder gemischt aliphatisch-cycloaliphatischer, gegebenenfalls durch Sauerstoffunktionen oder Halogenatome substituierter Kohlenwasserstoffrest mit 1 bis 21 C-Atomen ist, und R² tert.-Butyl, Methyl, Ethyl, Propyl oder Benzyl bedeutet, wobei die Verbindungen ausgenommen sind, in denen R ein Kohlenwasserstoffrest ist, R₁ = H, X = O, Y = O und R₂ = tert.-Butyl ist, dadurch gekennzeichnet, daß eine Verbindung der Formel II 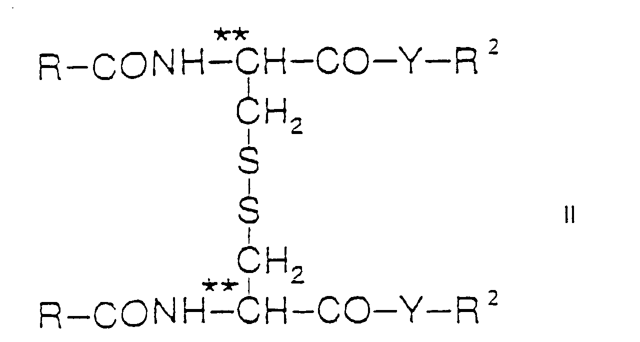 mit Zn in einem HCl/H₂SO₄/Lösungsmittel-Gemisch reduziert und mit einer Verbindung der Formel III 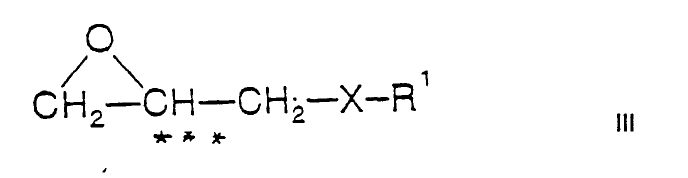 zu einer Verbindung der Formel I umgesetzt wird, wobei R, R¹, R², X und Y die obengenannten Bedeutungen haben. Die Epoxide der Formel III besitzen am mit *** gekennzeichnete Asymmetriezentrum die Absolutkonfiguration R oder S, und liegen enantiomerenrein oder gegebenenfalls als Racemat vor.

Besonders geeingnet ist das erfindungsgemäße Verfahren zur Herstellung von Verbindungen der Formel I, in denen R einen Fluorenylmethyloxy, tert.-Butyloxy-, einen gesättigten aliphatischen Kohlenwasserstoffrest mit 5 bis 17 C-Atomen bedeutet, X Sauerstoff oder Methylen (CH₂) und Y Sauerstoff bedeutet, R¹ Wasserstoff, einen gesättigten aliphatischen Kohlenwasserstoffrest mit 7 bis 18 C-Atomen oder einen Rest der Formel C(O)-R³ bedeutet, wobei R³ ein gesättigter Kohlenwasserstoffrest mit 5 bis 17 C-Atomen ist, und R² tert.-Butyl bedeutet. Bevorzugt sind weiter Verbindungen, die an den mit * bzw. ** bezeichneten Asymmetrie-Zentren die absolute R-Konfiguration besitzen.

Ganz besonders geeignet ist das Verfahren zur Herstellung von Verbindungen der Formel I, in denen R einen Fluorenylmethyloxy- oder einen gesättigten aliphatischen Kohlenwasserstoffrest mit 11 bis 17 C-Atomen bedeutet, X Sauerstoff und Y Sauerstoff bedeuten, R¹ Wasserstoff, einen gesättigten aliphatischen Kohlenwasserstoffrest mit 9 bis 18 C-Atomen oder einen Rest der Formel C(O)-R³ bedeutet, wobei R³ ein gesättigter Kohlenwasserstoffrest mit 9 bis 17 C-Atomen ist, und R² tert.-Butyl bedeutet. Ganz besonders bevorzugt sind Verbindungen, die an den mit * bzw. ** bezeichneten Asymmetrie-Zentren die absolute R-Konfiguration besitzen.

Besonders geeignet ist das erfindungsgemäße Verfahren zur Herstellung von Verbindungen der Formel I', mit R¹ = H und X = O.

Es wurde überraschenderweise gefunden, daß sich Bis-N-acylierte Cystin-di-ester unter den erfindungsgemäß genannten Bedingungen in einer "Eintopfreaktion" mit Zink und Säure reduzieren und ohne vorherige Isolierung der entstehenden Cystein-Derivate mit Epoxiden in hoher Ausbeute alkylieren lassen, auch wenn säurelabile Schutzgruppen wie z.B. tert.-Butylester anwesend sind. Die Reaktion kann in mehreren Stufen ausgeführt werden, findet aber vorzugsweise als Eintopfreaktion statt. Die Vorteile der beschriebenen Reaktion bestehen u.a. darin, daß keine Isolierung der Reduktionsprodukte nötig ist, daß sie unter milden Bedingungen abläuft, zu hohen Ausbeuten an diastereomerenreinen Produkten führt und daß die Aufarbeitung des Reaktionsansatzes sehr einfach ist.

Die Herstellung der Ausgangsstoffe der Formel II z.B. K.-H. Wiesmüller, W. Besser, G. Jung, Hoppe Seyler's Z. Physiol. Chem. 364 (1983) 593 beschrieben. Ausgangsstoffe der Formel II in der R einen Fluorenylmethyloxy-, tert.-Butyloxy-, Benzyloxy-Rest bedeuten lassen sich in bekannter Weise erhalten z.B. durch Umsetzung mit der entsprechenden Cystin-bis-ester mit Fluorenylmethyloxycarbonyl-N-hydroxysuccinimidester (Lapatsanis, L, Milias, G., Froussios, K., Kolovos, M. (1983) Synthesis, 671-673).

Die Verbindungen der Formel III sind als Racemat und enantiomerenrein käuflich erhältlich (z.B. Aldrich, Fluka, Merck).

Für das Zn/HCl (z.B. 32 %, d = 1,16)/H₂SO₄ (d = 1,84)/Lösungsmittelgemisch finden vorzugsweise folgende Lösungsmittel Verwendung: Methanol, Ethanol, n-Propanol, 2-Propanol, und n-Butanol, ganz besonders bevorzugt Methanol, Ethanol, 2-Propanol, insbesondere Methanol.

Das Volumenverhältnis der beiden Säurekomponenten HCl (32 %, d = 1,16) und H₂SO₄ (d = 1,84) liegt vorzugweise im Bereich 10:1 bis 2:1, ganz besonders bevorzugt 10:1 bis 5:1, insbesondere 7:1. Das Volumenverhältnis Säure-Komponenten/Lösungsmittel liegt zweckmäßigerweise im Bereich 1:100 bis 10:100, bevorzugt 1:100 bis 5:100, insbesondere 4:100.

Das Verhältnis der Äquivalente an Zink zur Verbindung der Formel II beträgt zweckmäßigerweise 5:1 bis 20:1, bevorzugt 5:1 bis 10:1, ganz besonders bevorzugt 5:1 bis 8:1.

Das Verhältnis der Äquivalente an Verbindung der Formel II zur Verbindung der Formel III beträgt zweckmäßigerweise 1:2 bis 1:40, bevorzugt 1:4 bis 1:20, ganz besonders bevorzugt 1:10.

Die Reaktionstemperatur liegt vorzugsweise im Bereich 20-70°C.

Die Reduktion der Verbindung der Formel II mit Zn und in situ Alkylierung mit einer Verbindung der Formel III wird bevorzugt unter folgenden Bedingungen durchgeführt: Das Verhältnis der Äquivalente an Zink zur Verbindung der Formel II beträgt 7:1, das Volumenverhältnis der beiden Säurekomponenten HCl (32 %, d = 1,16) und H₂SO₄ (d = 1,84) beträgt 7:1. Das Volumenverhältnis Säure-Komponenten/Lösungsmittel liegt vorzugsweise bei ca. 4:100. Als Lösungsmittel wird bevorzugt Methanol verwendet. Das Verhältnis der Äquivalente an der Verbindung der Formel II zur Verbindung der Formel III beträgt vorzugsweise 1:10. Die bevorzugte Reaktionstemperatur liegt bei ca. 45°C.

Anschließend erfolgt die Isolierung der Verbindung der Formel I, indem filtriert wird und das Filtrat am Rotationsverdampfer eingeengt wird. Lipophilere Verbindungen fallen beim Eingießen des eingeengten Filtrates in Wasser aus und können durch Filtration sehr einfach isoliert werden. Weniger lipophile Verbindungen und Verbindungen, die als Öl anfallen, werden durch eine schnelle Flash-Chromatographie an Kieselgel mit Chloroform als Fließmittel gereinigt.

Die erhaltenen N-Acyl-S-(2-hydroxyalkyl)-cysteine stellen Zwischenprodukte bei der Herstellung von Lipopeptiden dar, die als synthetischen Immunadjuvantien und synthetischen Impfstoffen sowie Stimulantien von B-Lymphozyten und Makrophagen Verwendung finden.
Besonders geeignet ist das Verfahren zur Herstellung von Zwischenprodukten bei der Synthese der Lipoaminosäure S-[2,3-bis(palmitoyloxy)-propyl]-cystein (Pam₃Cys-OH; K.-H. Wiesmüller, W. Bessler, G. Jung, Hoppe Seyler's Z. Physiol. Chem. 364 (1983) 593) sowie von Lipopeptiden, die diese Lipoaminosäure oder eine Analogon davon N-terminal enthalten. Letztere besitzen erhebliche Bedeutung bei der Entwicklung synthetischer Immunadjuvantien und synthetischer Vakzine.

Es besteht die Möglichkeit, C-terminale Schutzgruppen in den Verbindungen der Formel I (Y = Sauerstoff, X, R, R¹ und R² wie oben) mit an sich bekannten Verfahren der Peptidchemie (z.B. Behandlung mit Trifluoressigsäure zur Entfernung von tert.-Butyl oder Behandlung mit NaOH zur Verseifung von Methylestern) abzuspalten, und die so erhaltenen Verbindungen der Formel I (R² = Wasserstoff, Y = Sauerstoff, und X, R, R¹ wie oben) zum Aufbau von Lipopeptiden bevorzugt in Festphasenpeptidsynthesen zu verwenden. Festphasenpeptidsynthesen sind seit langem bekannt. Ebenso sind die zur verfahrensgemäßen Herstellung zum Aufbau der Peptidkette der neuen Lipopeptide zu verwendeten Aminosäuren bzw. Peptide bekannt oder können nach an sich bekannten Methoden hergestellt werden.

Die freie(n) Hydroxy-Gruppe(n) des harz-gebundenen N-Acyl-S-(2-hydroxyalkyl)-cysteinyl-Peptids läßt/lassen sich direkt am Harz mit Säuren verestern. Bevorzugt werden dazu Säurechloride der Formel IV 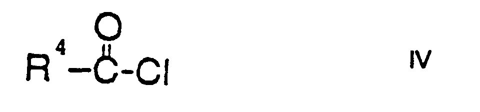 verwendet, wobei R⁴ einen bevorzugt gesättigten aber auch ungesättigten, aliphatischen oder gemischt aliphatisch-cycloaliphatischen, gegebenenfalls durch Sauerstoffunktionen oder Halogenatomen substituierten, Kohlenwasserstoffrest mit 1 bis 21 C-Atomen, bevorzugt mit 11 bis 19 und ganz besonders bevorzugt mit 11 bis 17 C-Atomen. Das Lösungsmittel für diese Acylierung ist ganz besonders bevorzugt Pyridin/Dichlormethan. (1:1; v:v).

Bei Verwendung von Verbindungen der Formel I mit R = Fluorenylmethyloxy, Y = Sauerstoff, R³ = Wasserstoff und X, R sowie R¹ wie oben beschrieben besteht die Möglichkeit nach Kupplung an ein harzgebundenes Peptid die basenlabile Amino-Schutzgruppe am N-Terminus Fluorenylmethyloxycarbonyl mit an sich bekannten Methoden (z.B. Behandlung mit Piperidin/DMF 1:1) abzuspalten. Der N-Terminus des harz-gebundenen Peptides kann dann mit geeigneten, weiteren Aminosäure-Derivaten unter üblichen Kupplungsbedingungen verlängert werden oder in Pyridin/Dichlormethan (1:1, v:v) mit Säurechloriden der Formel IV amidiert werden, wobei R⁴ die obengenannte Bedeutung besitzt.
Der entscheidende Vorteil der Durchführung der N- und O-Acylierung in zwei getrennten Schritten liegt in der Möglichkeit, auf einfache Weise Lipopeptide zu synthetisieren, die sich in der Art ihrer ester- und amidartig gebundenen Fettsäuren unterscheiden.

Die Abspaltung der harz-gebundenen Lipopeptide erfolgt auf an sich bekannte Weise, z.B. bei Verwendung säurelabiler Anker mit Trifluoressigsäure (siehe z.B. Metzger, J., Wiesmüller, K.-H., Schaude, R., Besser, W.G und Jung, G. (1991): Synthesis of Novel Immunologically Active Tripalmitoyl-S-glycerylcysteinyl Lipopeptides as Useful Intermediates for Immunogen Preparations, Int. J. Peptide Prot. Res. 37, 46-57 (1991)).

Folgende beispielhafte Verbindungen können nach obengenannten Herstellungsverfahren gemäß der Erfindung erhalten werden:
Nₐ-Fluorenylmethyloxycarbonyl-S-[2,3-dihydroxy-[2R]-propyl]-[R]-cysteine-tert.-butylester
Nₐ-Fluorenylmethyloxycarbonyl-S-[2,3-dihydroxy-[2S]-propyl]-[R]-cysteine-tert.-butylester
Nₐ-Fluorenylmethyloxycarbonyl-S-[2,3-dihydroxy-[2R,S]-propyl]-[R]-cysteine-tert.-butylester
N-Palmitoyl-S-(2[R,S]-hydroxy)-octadecyl-cystein-tert.-butylester
N-Palmitoyl-S-[2-hydroxy-3-palmitoyloxy[2R,S]-propyl]-cystein-tert.-butylester
N-Palmitoyl-S-[2-hydroxy-3-hexadecyloxy[2R,S]-propyl]-cystein-tert.-butylester
N-Palmitoyl-S-[2-hydroxy-3-butyloxy[2R,S]-propyl]-cystein-tert.-butylester
N-Palmitoyl-S-[2-hydroxy-3-methyloxy[2R,S]-propyl]-cystein-tert.-butylester

Die Hydroxy-Funktionen oben aufgeführter Verbindungen lassen sich mit entsprechenden Säurechloriden entweder in Lösung oder harzgebunden am Harz in Mischungen aus Pyridin und Dichlormethan (1:1, v:v) zu folgenden Verbindungen acylieren:
Nₐ-Fluorenylmethyloxycarbonyl-S-[2,3-bis(palmitoyloxy)-[2R]-propyl]-[R]-cystein-tert.-butylester
Nₐ-Fluorenylmethyloxycarbonyl-S-[2,3-bis(palmitoyloxy)-[2S]-propyl]-[R]-cystein-tert.-butylester
Nₐ-Fluorenylmethyloxycarbonyl-S-[2,3-bis(palmitoyloxy)-[2R,S]-propyl]-[R]-cystein-tert.-butylester
N-Palmitoyl-S-(2[R,S]-palmitoyloxy)-octadecyl-cystein-tert.-butylester
N-Palmitoyl-S-[2-lauroyloxy-3-palmitoyloxy-[2R,S]-propyl]-cystein-tert.-butylester
N-Palmitoyl-S-[2-lauroyloxy-3-hexadecyloxy[2R,S]-propyl]-cystein-tert.-butylester
N-Palmitoyl-S-[2-stearoyloxy-3-butyloxy[2R,S]-propyl]-cystein-tert.-butylester
N-Palmitoyl-S-[2-stearoyloxy-3-methyloxy[2R,S]-propyl]-cystein-tert.-butylester

Aus den entsprechenden oben aufgeführten tert.-Butylestern können durch Behandlung mit reiner Trifluoressigsäure folgende wichtige Aminosäure-Derivate erhalten werden:
Nₐ-Fluorenylmethyloxycarbonyl-S-[2,3-dihydroxy-[2R]-propyl]-[R]-cystein
Nₐ-Fluorenylmethyloxycarbonyl-S-[2,3-dihydroxy-[2S]-propyl]-[R]-cystein
Nₐ-Fluorenylmethyloxycarbonyl-S-[2,3-dihydroxy-[2R,S]-propyl]-[R,R]-cystein
Nₐ-Fluorenylmethyloxycarbonyl-S-[2,3-bis(palmitoyloxy)-[2R]-propyl]-[R]-cystein
Nₐ-Fluorenylmethyloxycarbonyl-S-[2,3-bis(palmitoyloxy)-[2S]-propyl]-[R]-cystein
Nₐ-Fluorenylmethyloxycarbonyl-S-[2,3-bis(palmitoyloxy)-[2R,S]-propyl]-[R]-cystein
N-Palmitoyl-S-(2[R,S]-hydroxy)-octadecyl-cystein
N-Palmitoyl-S-(2[R,S]-palmitoyloxy)octadecyl-cystein
N-Palmitoyl-S-[2-hydroxy-3-palmitoyloxy[2R,S]-propyl]-cystein
N-Palmitoyl-S-[2-hydroxy-3-hexadecyloxy[2R,S]-propyl]-cystein
N-Palmitoyl-S-[2-hydroxy-3-butyloxy[2R,S]-propyl]-cystein
N-Palmitoyl-S-[2-hydroxy-3-methyloxy[2R,S]-propyl]-cystein
N-Palmitoyl-S-[2-lauroyloxy-3-palmitoyloxy[2R,S]-propyl]-cystein
N-Palmitoyl-S-[2-lauroyloxy-3-hexadecyloxy[2R,S]-propyl]-cystein
N-Palmitoyl-S-[2-stearoyloxy-3-butyloxy[2R,S]-propyl]-cystein
N-Palmitoyl-S-[2-stearoyloxy-3-methyloxy[2R,S]-propyl]-cystein

Die vorliegende Erfindung, die auch durch den Inhalt der Patentansprüche charaktierisert ist soll durch die nachfolgenden Ausführungsbeispiele näher erläutert werden:

Alle nachfolgend genannten Beispielsverbindungen wurden mit Ion Spray Massenspektrometrie IS-MS und IS-MS/MS untersucht. Die Verbindungen konnten sowohl über ihre Molmasse als auch über ihre Fragmentierung eindeutig charakterisiert werden.

Im DC verwendet man Kieselgel als Adsorbens und folgende Systeme als Laufmittel:
I, Chloroform/Methanol/Wasser (65:25:4); II, Chloroform/Methanol/Eisessig (90:10:1); III, Ethylacetat gesättigt mit Wasser; IV, 1-Butanol/Eisessig/Wasser (2:1:1); V, Chloroform/Methanol (8:2); VI, Chloroform/Methanol/17 % Ammoniak (2:2:1). VII, Chloroform. Zur Detektion wurden die Platten mit Wasser, Ninhydrin Reagenz und Chlor/4,4'-bis(dimethylamino)-diphenylmethane (TDM Reagenz) besprüht.

### Beispiel 1

### Nₐ-Fluorenylmethyloxycarbonyl-S-[2,3-dihydroxy-propyl]-[R]-cystein-tert.-butylester

Zu einer Losung von N,N'-Dipalmitoyl-cystin-di-tert.-butylester (1,92 g; 2,4 mmol) in Dichlormethan (15 ml) wurde Zink (1,1 g; 16,8 mmol) und eine frisch bereitete Lösung von Methanol, 32 % Salzsäure (d = 1,16) und konzentrierter Schwefelsäure (d = 1,84) (100:7:1; 8 ml) unter starkem Rühren zugegeben. Nach 15 min wurde (S)-(-)-Glycidol (1,78 g = 1,6 ml; 2,4 mmol) zugegeben. Die Mischung wurde 5 h bei 40°C gerührt. Das Lösungsmittel wurde auf etwa die Hälfte des ursprünglichen Volumens eingeengt und mit 5 % KHSO₄ (2 ml) verdünnt. Diese Mischung wurde 16 h lang bei -4°C aufbewahrt und 3 x mit Dichlormethan ausgeschüttelt. Die organische Phase wurde über Na₂SO₄ getrocknet und zur Trockne eingeengt. Der Ester wurde als farbloses Öl erhalten. Ausbeute für das RR-Diastereomer: 2,06 g (91 %). R_{f}(I) = 0,72; R_{f}(II) = 0,47; R_{f}(IV) = 0,45; C₂₅H₃₁N O₆S (473,6). Analog wurden unter Verwendung von (R)-(+ )-Glycidol das RS-Diastereomer (Ausbeute 1,93 g; 85 %) und mit racemischem Glycidol die Mischung R(R/S) aus beiden Diastereomeren erhalten. ¹³C-NMR: siehe nachfolgende Tabelle.

Zuordnung der ¹³C-NMR Signale für Nₐ-Fluorenylmethoxycarbonyl-S-[2,3-dihydroxy-propyl]-cystein-tert.-butylester (CDCl₃; 200 mg/ml; 20,115 MHz):

| | RR | RS | R(R/S) |
|---|---|---|---|
| t-Bu-CH₃ | 27,9 | 27,9 | 27,9 |
| S-Glyceryl CH₂ | 35,5 | 35,4 | 35,4/35,5 |
| Cys-C_{B} | 36,2 | 36,3 | 36,2/36,3 |
| Fmoc-C-9 | 47,1 | 47,0 | 47,0 |
| Cys-C_{α} | 54,6 | 54,5 | 54,5 |
| S-Glyceryl CH₂-OH | 65,1 | 65,1 | 65,0/65,0 |
| Fmoc-CH₂-O | 67,2 | 67,2 | 67,1 |
| S-Glyceryl CH-OH | 71,0 | 71,1 | 71,0/71,0 |
| Tbu-C_{q} | 83,0 | 82,9 | 82,9 |
| Fmoc-C-4,5 | 120,0 | 119,9 | 119,9 |
| Fmoc-C-1,8 | 125,1 | 125,1 | 125,1 |
| Fmoc-C-3,2,6,7 | 127,1 | 127,1 | 127,0 |
| Fmoc-C-3,2,6,7 | 127,7 | 127,7 | 127,7 |
| Fmoc-C-4a,4b | 141,2 | 141,2 | 141,2 |
| Fmoc-C-8a,9a | 143,7 | 143,7 | 143,7 |
| Fmoc-CO | 156,2 | 156,1 | 156,1 |
| Cys-CO | 169,9 | 169,9 | 169,3 |

Der in der Vorstufe verwendete N,N'-Bis(fluorenylmethoxycarbonyl)-[R]-cystin-di-tert.-butylester wurde nach folgender Vorschrift hergestellt: L-Cystin-di-tert.-butylester (3,52 g; 10 mmol) und Fluorenylmethoxycarbonyl-N-hydroxysuccinimidester (5,4 g; 16 mmol) wurden in Tetrahydrofuran (10 ml) gelöst. Eine Lösung von N-Ethylmorpholin (2,55 g; 20 mmol) in Tetrahydrofuran (5 ml) wurde zugegeben. Nach 3 h wurde die Mischung zur Trockne eingeengt. Der Rückstand wurde in Essigester gelöst und 3 x mit 5 % KHSO₄ und Wasser gewaschen. Die organische Phase wurde über Na₂SO₄ getrocknet und zur Trockne eingeengt. Der Rückstand wurde aus Dichlormethan/Methanol (1:4; 300 ml) bei - 20°C umkristallisiert. Die Fällung wurde 2 x mit tert.-Butanol/2-Propanol (1:1) gewaschen und über P₄O₁₀/KOH getrocknet. Ausbeute: 6,9 g (87 %). R_{f}(II) = 0,89; R_{f}(III) = 0,81; R_{f}(IV) = 0,80; R_{f}(V) = 0,21.
C₄₄H₄₈N₂O₈S₂ (797,0). Ion Spray MS [M+H]⁺ 798.
¹³C-NMR (CDCl₃; 150 mg/ml; 120 scans; 20,115 MHz): δ(ppm) = 27,9(3 x tBu-CH₃); 41,8 (Cys-C_{B}); 47,0 (Fmoc-C-9); 54,1 (Cys-C_{α}); 67,2 (Fmoc-CH₂-O); 83,0 (Cys-OtBu-C_{q}); 119,9 (Fmoc-C-4,5); 125,1 (Fmoc-C-1,8); 127,6, 127,0 (Fmoc-C-3,2,6,7); 141,2 (Fmoc-C-4a,4b); 143,8, 143,7 (Fmoc-C-8a,9a); 155,7 (Fmoc-CO); 169,3 (Cys-CO).

Der in der Vorstufe verwendete L-Cystin-di-tert.-butylester wurde nach einer bekannten Literaturvorschrift hergestellt (K.-H. Wiesmüller, W. Bessler, G. Jung, Hoppe Seyler's Z. Physiol. Chem. 364 (1983) 593).

### Beispiel 2

### N-Palmitoyl-S-(2-[R,S]-hydroxy)-octadecyl-cystein-tert.-butylester

N,N'-Dipalmitoyl-cystin-di-tert.-butylester (2 g; 2,42 mMol) wurde unter leichtem Erwärmen in Methanol (15 ml) gelöst und mit Zinkstaub (1,1 g; 16,8 mMol) versetzt. Über einen Tropftrichter wurde eine Mischung (7:1; 1 ml) aus konz. HCl (32 %, d = 1,16) und konz. Schwefelsäure (d = 1,84) unter starkem Rühren zugetropft. Nach 15 min wurde 1,2-Epoxy-octadecan (1,3 g; 4,84 mMol) zugegeben. Es wurde 10 h bei 45°C gerührt. Das Lösungsmittel wurde nach Beendigung der Reaktion am Rotationsverdampfer entfernt, das Produkt wurde aus Chloroform bei -20°C gefällt. Zur weiteren Reinigung wurde an Kieselgel chromatographiert (Säule 80 x 2,5 cm; Eluent: Chloroform). Die Produktfraktionen werden vereint und durch eine weitere Fallung aus Methanol bei 4°C als farbloses Pulver in hoher Reinheit erhalten. Ausbeute: 2,4 g (73 %).
C₄₁H₈₁NO₄S 684,2; Ion Spray MS und MS/MS: siehe Fig. 1. R_{f}(VII) = 0,41.

### Beispiel 3

### N-Palmitoyl-S-[2-hydroxy-3-palmitoyloxy-[2R,S]-propyl]-cystein-tert.butylester

N,N'-Dipalmitoyl-cystin-di-tert.-butylester (1 g; 1,21 Mmol) wurde unter leichtem Erwärmen in Methanol (10 ml) gelöst und mit Zinkstaub (550 mg; 8,5 Mmol) versetzt. Über einen Tropftrichter wurde eine Mischung (7:1; 0,5 ml) aus konz. Hcl (32 %, d = 1,16) und konz. Schwefelsäure (d = 1,84) unter starkem Rühren zugetropft. Nach 20 min wurde Palmitinsäure-2,3-epoxy-1-propylester (1,62 g; 4,84 Mmol) zugegeben. Es wurde 10 h bei 45°C gerührt. Das Lösungsmittel wurde nach Beendigung der Reaktion am Rotationsverdampfer entfernt, das Produkt wurde aus Methanol/Eisessig/Wasser (5:1:1) bei -20°C gefällt und aus tert.-Butanol lyophilisiert. Eine weitere Fällung aus Methanol bei 4°C lieferte den Lipoaminosäureester in hoher Reinheit. Ausbeute: 1,25 g (71 %). C₄₂H₈₁NO₆S 728,2; Ion Spray MS: [M+H]⁺ 729; R_{f}(VI) = 0,34.

Der oben verwendete N,N'-Dipalmitoyl-cystin-di-tert.-butylester wurde nach Literaturvorschrift hergestellt (K.-H. Wiesmüller, W. Bessler, G. Jung, Hoppe Seyler's Z. Physiol. Chem. 364 (1983) 593); das gilt auch für Palmitinsäure-2,3-epoxy-1-propylester (Kester et al., J. Org. Chem. 8, 553, 1943).

### Beispiel 4

### N-Palmitoyl-S-[2-hydroxy-3-butyloxy-[2R,S]-propyl]-cystein-tert.butylester

N,N'-Dipalmitoyl-cystin-di-tert.butylester (1 g; 1,21 mMol) wurde unter leichtem Erwärmen in Methanol (10 ml) gelöst und mit Zinkstaub (280 mg; 4,3 mMol) versetzt. Über einen Tropftrichter wurde eine Mischung (7:1; 0,5 ml) aus konz. HCl (32 %, d = 1,16) und konz. Schwefelsäure (d = 1,84) unter starkem Rühren zugetropft. Nach 10 min wurde Glycidylbutylether (0,63 g; 4,84 mMol) zugegeben. Es wurde 5 h bei 45°C gerührt. Das Lösungsmittel wurde nach Beendigung der Reaktion am Rotationsverdampfer entfernt. Zur Reinigung wurde an Kieselgel chromatographiert (Säule 80 x 2,5 cm; Eluent: Chloroform). Die Produktfraktionen wurden vereint, zur Trockne eingeengt und das resultierende farblose Öl am Hochvakuum getrocknet. Ausbeute: 0,95 g (72 %). C₃₀H₅₉NO₅ 545,9; Ion Spray MS: [M+H]⁺ 547; R_{f}(III) = 0,34.

Der oben verwendete N,N'-Dipalmitoyl-cystin-di-tert.-butylester wurde nach Literaturvorschrift hergestellt (K.-H. Wiesmüller, W. Bessler, G. Jung, Hoppe Seyler's Z. Physiol. Chem. 364 (1983) 593).

## Patentansprüche (Patentansprüche für folgende(n) Vertragsstaat(en): AT, BE, CH, DE, DK, FR, GB, IT, LI, LU, MC, NL, PT, SE)

1. Verbindungen der Formel I 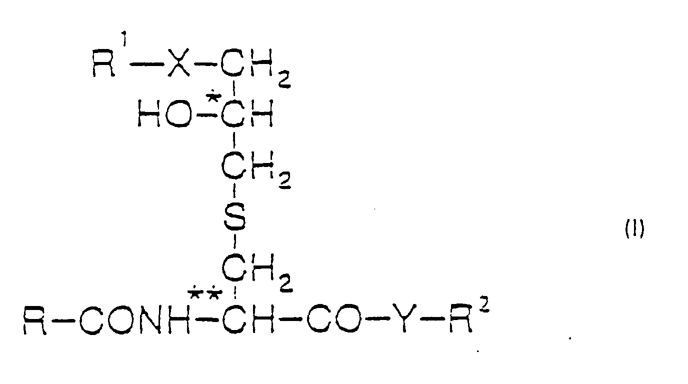 in der R einen Fluorenylmethyloxyrest, X Sauerstoff, Y Sauerstoff, R¹ Wasserstoff, einen gesättigten aliphatischen Kohlenwasserstoffrest mit 9 bis 18 C-Atomen oder einen Rest der Formel C(O)-R³ bedeutet, wobei R³ ein gesättigter Kohlenwasserstoffrest mit 9 bis 17 C-Atomen ist, und R² tert. Butyl bedeutet.

2. Verfahren zur Herstellung von Verbindungen der Formel I 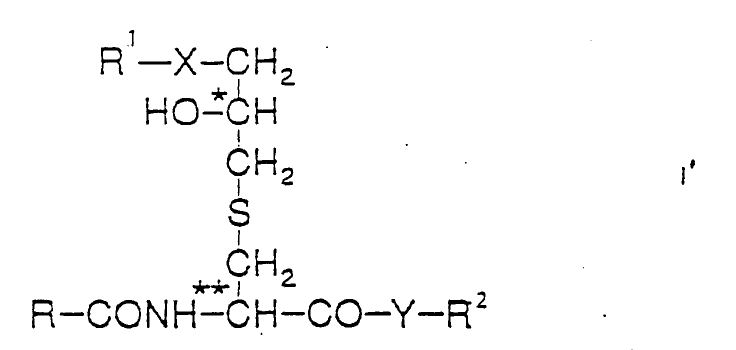 in der R einen Fluorenylmethyloxy-, tert.-Butyloxy-, Benzyloxy-Rest oder einen gesättigten oder ungesättigten, aliphatischen oder gemischt aliphatisch-cycloaliphatischen, gegebenenfalls durch Sauerstoffunktionen oder Halogenatome substituierten Kohlenwasserstoffrest mit 1 bis 21 C-Atomen bedeutet, X Sauerstoff (O) oder Methylen (CH₂) bedeutet, Y Sauerstoff oder ein gegebenenfalls mit einer in der Peptidchemie üblichen Schutzgruppen seitenkettengeschützter Aminosäurerest ist, R¹ Wasserstoff, einen gesättigten oder ungesättigten, aliphatischen oder gemischt aliphatisch-cycloaliphatischen, gegebenenfalls durch Sauerstoffunktionen oder Halogenatome substituierten Kohlenwasserstoffrest mit 1 bis 21 C-Atomen oder einen Rest der Formel C(O)-R³ bedeutet, wobei R³ ein gesättigter oder ungesättigter, aliphatischer oder gemischt aliphatisch-cycloaliphatischer, gegebenenfalls durch Sauerstoffunktionen oder Halogenatome substituierter Kohlenwasserstoffrest mit 1 bis 21 C-Atomen ist, und R² tert.-Butyl, Methyl, Ethyl, Propyl oder Benzyl bedeutet, wobei die Verbindungen ausgenommen sind, in denen R ein Kohlenwasserstoffrest ist, R₁ = H, X = O, Y = O und R₂ = tert.-Butyl ist, dadurch gekennzeichnet, daß eine Verbindung der Formel II 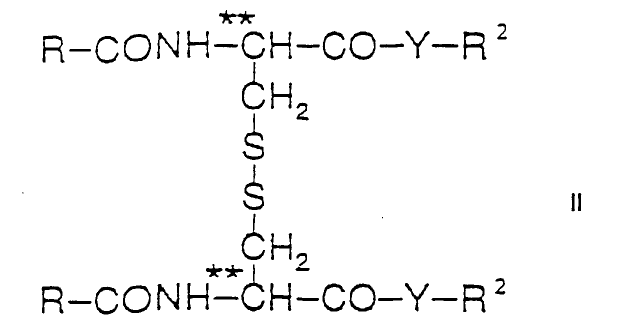 mit Zn in einem HCl/H₂SO₄/Lösungsmittel-Gemisch reduziert und mit einer Verbindung der Formel III 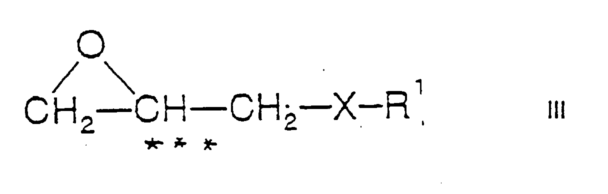 umgesetzt wird, wobei R, R¹, R², X und Y die obengenannten Bedeutungen haben.

3. Verfahren zur Herstellung von Verbindungen der Formel I' gemäß Anspruch 2, dadurch gekennzeichnet, daß R einen Fluorenylmethyloxy-, tert.-Butyloxy-, einen gesättigten aliphatischen Kohlenwasserstoffrest mit 5 bis 17 C-Atomen bedeutet, X Sauerstoff oder Methylen (CH₂) und Y Sauerstoff bedeutet, R¹ Wasserstoff, einen gesättigten aliphatischen Kohlenwasserstoffrest mit 7 bis 18 C-Atomen oder einen Rest der Formel C(O)-R³ bedeutet, wobei R³ ein gesättigter Kohlenwasserstoffrest mit 5 bis 17 C-Atomen ist, und R² tert.-Butyl bedeutet, vorzugsweise mit absoluter R-Konfiguration an den mit * bzw. ** bezeichneten Asymmetrie-Zentren.

4. Verfahren zur Herstellung von Verbindungen der Formel I', gemäß den Ansprüchen 2 oder 3, dadurch gekennzeichnet, daß R einen Fluorenylmethyloxy- oder einen gesättigten aliphatischen Kohlenwasserstoffrest mit 11 bis 17 C-Atomen bedeutet, X Sauerstoff und Y Sauerstoff bedeuten, R¹ Wasserstoff, einen gesättigten aliphatischen Kohlenwasserstoffrest mit 9 bis 18 C-Atomen oder einen Rest der Formel C(O)-R³ bedeutet, wobei R³ ein gesättigter Kohlenwasserstoffrest mit 9 bis 17 C-Atomen ist, und R² tert.-Butyl bedeutet, vorzugsweise mit absoluter R-Konfiguration an den mit * bzw. ** bezeichneten Asymmetrie-Zentren.

5. Verfahren gemäß den Ansprüchen 2, 3 oder 4, dadurch gekennzeichnet, daß es als Eintopfreaktion durchgeführt wird.

6. Verwendung von Verbindungen der Formel I gemäß Anspruch 1 zur Herstellung von Lipopeptiden und Lipoaminosäuren.

7. Verwendung von Verbindungen der Formel I gemäß Anspruch 1 zur Herstellung von Pam₃CysOH und Pam₃Cys-haltigen Peptiden.

8. Verwendung von Verbindungen der Formel I gemäß Anspruch 1 zum Aufbau von Lipopeptiden in der Festphasensynthese nach Abspaltung der entsprechenden Säureschutzgruppen.

9. Verwendung von Verbindungen der Formel I gemäß Anspruch 1 in der Festphasensynthese gemäß Anspruch 8 dadurch gekennzeichnet, daß freie Hydroxygruppen des harzgebundenen Peptides mit Säurechloriden der Formel IV 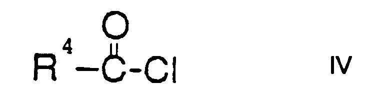 wobei R⁴ einen Kohlenwasserstoffrest mit 1 bis 21 C-Atomen bedeutet, verestert werden.

## Patentansprüche (Patentansprüche für folgende(n) Vertragsstaat(en): ES, GR)

1. Verfahren zur Herstellung von Verbindungen der Formel I 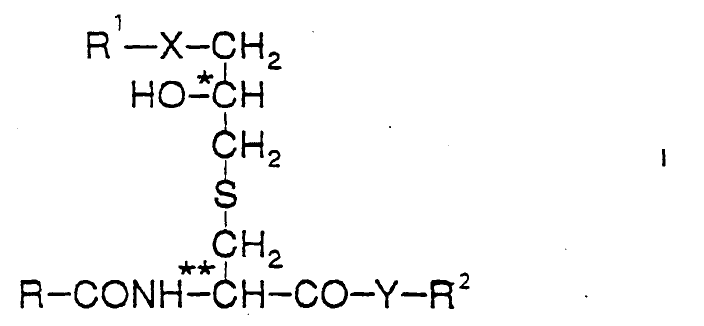 in der R einen Fluorenylmethyloxy-, tert.-Butyloxy-, Benzyloxy-Rest oder einen gesättigten oder ungesättigten, aliphatischen oder gemischt aliphatisch-cycloaliphatischen, gegebenenfalls durch Sauerstoffunktionen oder Halogenatome substituierten Kohlenwasserstoffrest mit 1 bis 21 C-Atomen bedeutet, X Sauerstoff (O) oder Methylen (CH₂) bedeutet, Y Sauerstoff oder ein gegebenenfalls mit einer in der Peptidchemie üblichen Schutzgruppen seitenkettengeschützter Aminosäurerest ist, R¹ Wasserstoff, einen gesättigten oder ungesättigten, aliphatischen oder gemischt aliphatisch-cycloaliphatischen, gegebenenfalls durch Sauerstoffunktionen oder Halogenatome substituierten Kohlenwasserstoffrest mit 1 bis 21 C-Atomen oder einen Rest der Formel C(O)-R³ bedeutet, wobei R³ ein gesättigter oder ungesättigter, aliphatischer oder gemischt aliphatisch-cycloaliphatischer, gegebenenfalls durch Sauerstoffunktionen oder Halogenatome substituierter Kohlenwasserstoffrest mit 1 bis 21 C-Atomen ist, und R² tert.-Butyl, Methyl, Ethyl, Propyl oder Benzyl bedeutet, wobei die Verbindungen ausgenommen sind, in denen R ein Kohlenwasserstoffrest ist, R₁ = H, X = O, Y = O und R₂ = tert.-Butyl ist, dadurch gekennzeichnet, daß eine Verbindung der Formel II 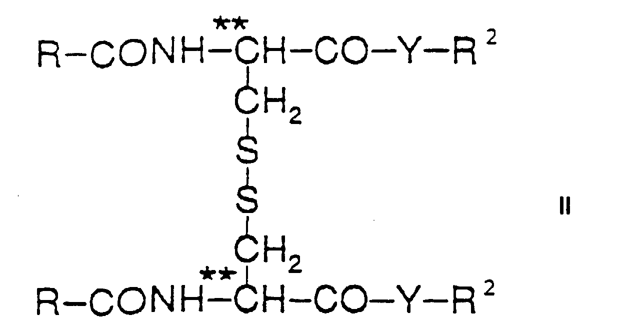 mit Zn in einem HCl/H₂SO₄/Lösungsmittel-Gemisch reduziert und mit einer Verbindung der Formel III 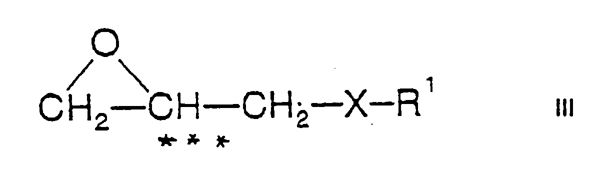 umgesetzt wird, wobei R, R¹, R², X und Y die obengenannten Bedeutungen haben.

2. Verfahren zur Herstellung von Verbindungen der Formel I gemäß Anspruch 1, dadurch gekennzeichnet, daß R einen Fluorenylmethyloxy-, tert.-Butyloxy-, einen gesättigten aliphatischen Kohlenwasserstoffrest mit 5 bis 17 C-Atomen bedeutet, X Sauerstoff oder Methylen (CH₂) und Y Sauerstoff bedeutet, R¹ Wasserstoff, einen gesättigten aliphatischen Kohlenwasserstoffrest mit 7 bis 18 C-Atomen oder einen Rest der Formel C(O)-R³ bedeutet, wobei R³ ein gesättigter Kohlenwasserstoffrest mit 5 bis 17 C-Atomen ist, und R² tert.-Butyl bedeutet, vorzugsweise mit absoluter R-Konfiguration an den mit * bzw. ** bezeichneten Asymmetrie-Zentren.

3. Verfahren zur Herstellung von Verbindungen der Formel I, gemäß den Ansprüchen 1 oder 2, dadurch gekennzeichnet, daß R einen Fluorenylmethyloxy- oder einen gesättigten aliphatischen Kohlenwasserstoffrest mit 11 bis 17 C-Atomen bedeutet, X Sauerstoff und Y Sauerstoff bedeuten, R¹ Wasserstoff, einen gesättigten aliphatischen Kohlenwasserstoffrest mit 9 bis 18 C-Atomen oder einen Rest der Formel C(O)-R³ bedeutet, wobei R³ ein gesättigter Kohlenwasserstoffrest mit 9 bis 17 C-Atomen ist, und R² tert.-Butyl bedeutet, vorzugsweise mit absoluter R-Konfiguration an den mit * bzw. ** bezeichneten Asymmetrie-Zentren.

4. Verfahren zur Herstellung von Verbindungen der Formel I gemäß einem oder mehreren der Ansprüche 1 bis 3, dadurch gekennzeichnet, daß R einen Fluorenylmethyloxyrest, X Sauerstoff, Y Sauerstoff, R¹ Wasserstoff, einen gesättigten aliphatischen Kohlenwasserstoffrest mit 9 bis 18 C-Atomen oder einen Rest der Formel C(O)-R³ bedeutet, wobei R³ ein gesättigter Kohlenwasserstoffrest mit 9 bis 17 C-Atomen ist, und R² tert. Butyl bedeutet.

5. Verfahren gemäß Anspruch 1, dadurch gekennzeichnet, daß es als Eintopfreaktion durchgeführt wird.

6. Verwendung von Verbindungen der Formel I gemäß Anspruch 4 zur Herstellung von Lipopeptiden und Lipoaminosäuren.

7. Verwendung von Verbindungen der Formel I gemäß Anspruch 4 zur Herstellung von Pam₃CysOH und Pam₃Cys-haltigen Peptiden.

8. Verwendung von Verbindungen der Formel I gemäß Anspruch 4 zum Aufbau von Lipopeptiden in der Festphasensynthese nach Abspaltung der entsprechenden Säureschutzgruppen.

9. Verwendung von Verbindungen der Formel I gemäß Anspruch 4 in der Festphasensynthese gemäß Anspruch 8, dadurch gekennzeichnet, daß freie Hydroxygruppen des harzgebundenen Peptides mit Säurechloriden der Formel IV 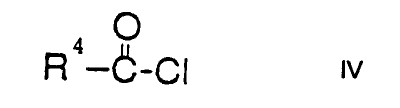 wobei R⁴ einen Kohlenwasserstoffrest mit 1 bis 21 C-Atomen bedeutet, verestert werden.

## Claims (Claims for the following Contracting State(s): AT, BE, CH, DE, DK, FR, GB, IT, LI, LU, MC, NL, PT, SE)

1. A compound of the formula I 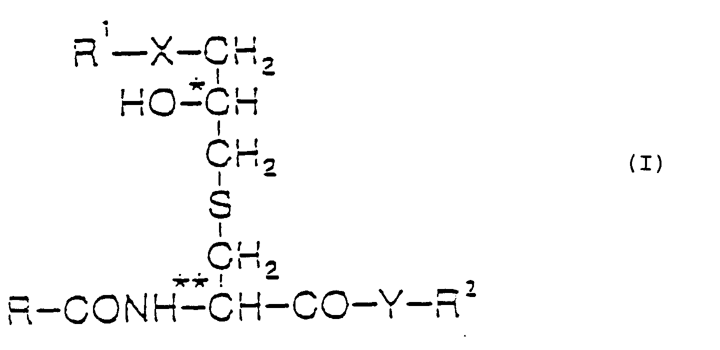 in which R is a fluorenylmethoxy radical, X is oxygen, Y is oxygen, R¹ is hydrogen, a saturated aliphatic hydrocarbon radical having 9 to 18 carbon atoms or a radical of the formula C(O)-R³, where R³ is a saturated hydrocarbon radical having 9 to 17 carbon atoms, and R² is tert-butyl.

2. A process for the preparation of a compound of the formula I' 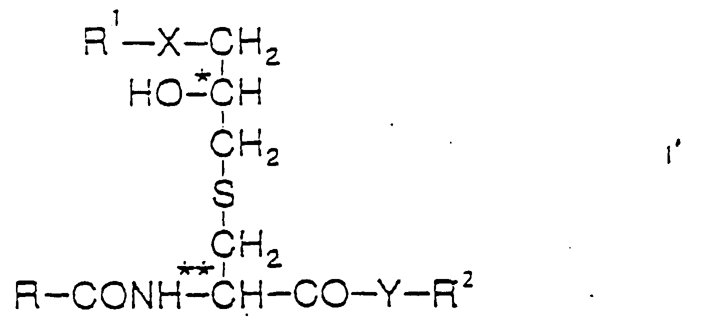 in which R is a fluorenylmethoxy, tert-butoxy or benzyloxy radical or a saturated or unsaturated, aliphatic or mixed aliphatic/cycloaliphatic hydrocarbon radical having 1 to 21 carbon atoms and optionally substituted by oxygen functions or halogen atoms, X is oxygen (O) or methylene (CH₂), Y is oxygen or an amino acid radical optionally side-chain-protected by a protective group customary in peptide chemistry, R¹ is hydrogen, a saturated or unsaturated, aliphatic or mixed aliphatic/cycloaliphatic hydrocarbon radical having 1 to 21 carbon atoms and optionally substituted by oxygen functions or halogen atoms, or a radical of the formula C(O)-R³, where R³ is a saturated or unsaturated, aliphatic or mixed aliphatic/cycloaliphatic hydrocarbon radical having 1 to 21 carbon atoms and optionally substituted by oxygen functions or halogen atoms, and R² is tert-butyl, methyl, ethyl, propyl or benzyl, where the compounds are excluded in which R is a hydrocarbon radical, R₁ = H, X = O, Y = O and R₂ = tert-butyl, which comprises reducing a compound of the formula II 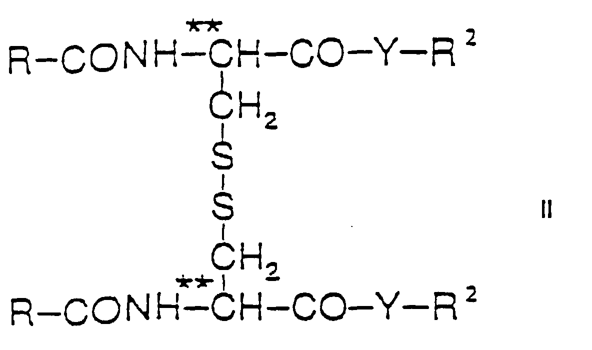 with Zn in an HCl/H₂SO₄/solvent mixture and reacting with a compound of the formula III 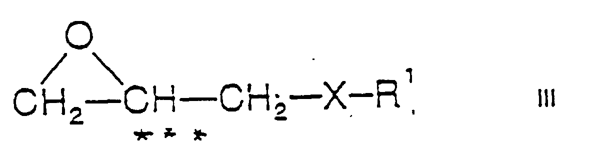 where R, R¹, R², X and Y have the abovementioned meanings.

3. A process for the preparation of a compound of the formula I' as claimed in claim 2, wherein R is a fluorenylmethoxy, tert-butoxy or a saturated aliphatic hydrocarbon radical having 5 to 17 carbon atoms, X is oxygen or methylene (CH₂) and Y is oxygen, R¹ is hydrogen, a saturated aliphatic hydrocarbon radical having 7 to 18 carbon atoms or a radical of the formula C(O)-R³, where R³ is a saturated hydrocarbon radical having 5 to 17 carbon atoms, and R² is tert-butyl, preferably with absolute R-configuration at the centers of asymmetry designated by * or **.

4. A process for the preparation of a compound of the formula I', as claimed in claims 2 or 3, wherein R is a fluorenylmethoxy or a saturated aliphatic hydrocarbon radical having 11 to 17 carbon atoms, X is oxygen and Y is oxygen, R¹ is hydrogen, a saturated aliphatic hydrocarbon radical having 9 to 18 carbon atoms or a radical of the formula C(O)-R³, where R³ is a saturated hydrocarbon radical having 9 to 17 carbon atoms, and R² is tert-butyl, preferably with absolute R-configuration at the centers of asymmetry designated by * or **.

5. The process as claimed in claims 2, 3 or 4, which is carried out as a one-pot reaction.

6. The use of compounds of the formula I as claimed in claim 1 for the preparation of lipopeptides and lipoamino acids.

7. The use of compounds of the formula I as claimed in claim 1 for the preparation of Pam₃CysOH and Pam₃Cys-containing peptides.

8. The use of compounds of the formula I as claimed in claim 1 for the synthesis of lipopeptides in solid phase synthesis after removal of the corresponding acid protective groups.

9. The use of compounds of the formula I as claimed in claim 1 in solid phase synthesis as claimed in claim 8, wherein free hydroxyl groups of the resin-bonded peptide are esterified with acid chlorides of the formula IV 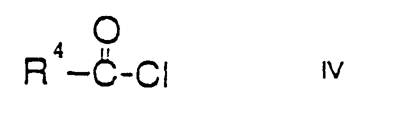 where R⁴ is a hydrocarbon radical having 1 to 21 carbon atoms.

## Claims (Claims for the following Contracting State(s): ES, GR)

1. A process for the preparation of a compound of the formula I 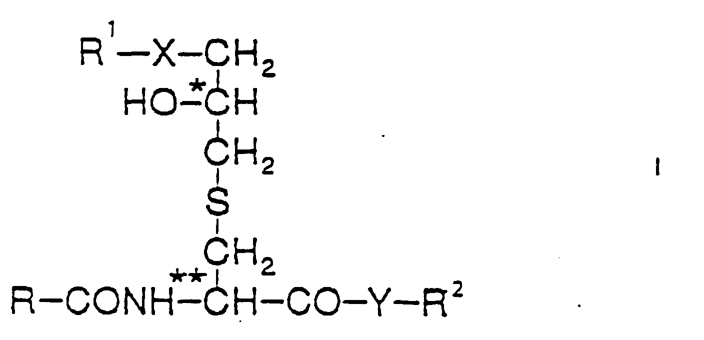 in which R is a fluorenylmethoxy, tert-butoxy or benzyloxy radical or a saturated or unsaturated, aliphatic or mixed aliphatic/cycloaliphatic hydrocarbon radical having 1 to 21 carbon atoms and optionally substituted by oxygen functions or halogen atoms, X is oxygen (O) or methylene (CH₂), Y is oxygen or an amino acid radical optionally side-chain-protected by a protective group customary in peptide chemistry, R¹ is hydrogen, a saturated or unsaturated, aliphatic or mixed aliphatic/cycloaliphatic hydrocarbon radical having 1 to 21 carbon atoms and optionally substituted by oxygen functions or halogen atoms, or a radical of the formula C(O)-R³, where R³ is a saturated or unsaturated, aliphatic or mixed aliphatic/cycloaliphatic hydrocarbon radical having 1 to 21 carbon atoms and optionally substituted by oxygen functions or halogen atoms, and R² is tert-butyl, methyl, ethyl, propyl or benzyl, where the compounds are excluded in which R is a hydrocarbon radical R₁ = H, X = O, Y = O and R₂ = tert-butyl, which comprises reducing a compound of the formula II 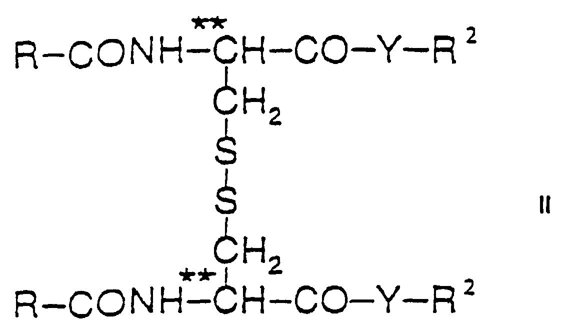 with Zn in an HCl/H₂SO₄/solvent mixture and reacting with a compound of the formula III 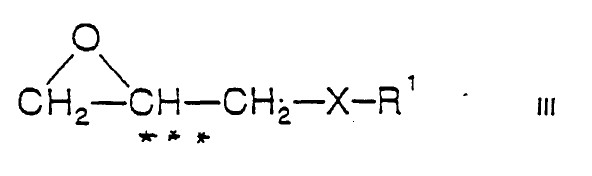 where R, R¹, R², X and Y have the abovementioned meanings.

2. A process for the preparation of a compound of the formula I as claimed in claim 1, wherein R is a fluorenylmethoxy, tert-butoxy or a saturated aliphatic hydrocarbon radical having 5 to 17 carbon atoms, X is oxygen or methylene (CH₂) and Y is oxygen, R¹ is hydrogen, a saturated aliphatic hydrocarbon radical having 7 to 18 carbon atoms or a radical of the formula C(O)-R³, where R³ is a saturated hydrocarbon radical having 5 to 17 carbon atoms, and R² is tert-butyl, preferably with absolute R-configuration at the centers of asymmetry designated by * or **.

3. A process for the preparation of a compound of the formula I, as claimed in claims 1 or 2, wherein R is a fluorenylmethoxy or a saturated aliphatic hydrocarbon radical having 11 to 17 carbon atoms, X is oxygen and Y is oxygen, R¹ is hydrogen, a saturated aliphatic hydrocarbon radical having 9 to 18 carbon atoms or a radical of the formula C(O)-R³, where R³ is a saturated hydrocarbon radical having 9 to 17 carbon atoms, and R² is tert-butyl, preferably with absolute R-configuration at the centers of asymmetry designated by * or **.

4. A process for the preparation of a compound of the formula I as claimed in one or more of claims 1 to 3, wherein R is a fluorenylmethoxy radical, X is oxygen, Y is oxygen, R¹ is hydrogen, a saturated aliphatic hydrocarbon radical having 9 to 18 carbon atoms or a radical of the formula C(O)-R³, where R³ is a saturated hydrocarbon radical having 9 to 17 carbon atoms, and R² is tert-butyl.

5. The process as claimed in claim 1, which is carried out as a one-pot reaction.

6. The use of compounds of the formula I as claimed in claim 4 for the preparation of lipopeptides and lipoamino acids.

7. The use of compounds of the formula I as claimed in claim 4 for the preparation of Pam₃CysOH and Pam₃Cys-containing peptides.

8. The use of compounds of the formula I as claimed in claim 4 for the synthesis of lipopeptides in solid phase synthesis after removal of the corresponding acid protective groups.

9. The use of compounds of the formula I as claimed in claim 4 in solid phase synthesis as claimed in claim 8, wherein free hydroxyl groups of the resin-bonded peptide are esterified with acid chlorides of the formula IV 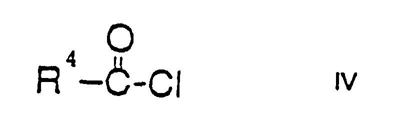 where R⁴ is a hydrocarbon radical having 1 to 21 carbon atoms.

## Revendications (Revendications pour l'(les) Etat(s) contractant(s) suivant(s): AT, BE, CH, DE, DK, FR, GB, IT, LI, LU, MC, NL, PT, SE)

1. Composés de formule I 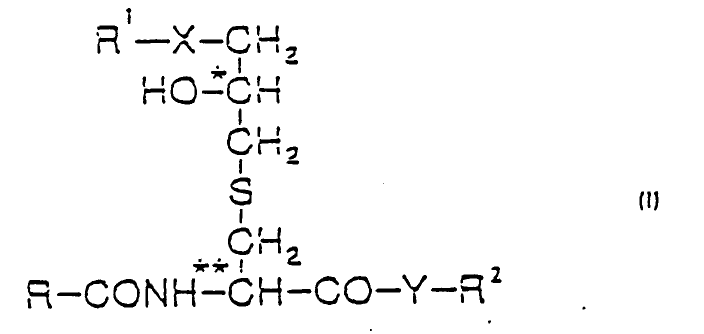 dans laquelle R représente le radical fluorénylméthyloxy, X représente un atome d'oxygène, Y représente un atome d'oxygène, R¹ représente un atome d'hydrogène, un radical hydrocarboné aliphatique saturé ayant de 9 à 18 atomes de carbone ou un radical de formule C(O)-R³, R³ étant un radical hydrocarboné saturé ayant de 9 à 17 atomes de carbone, et R² représente le groupe tert-butyle.

2. Procédé pour la préparation des composés de formule I 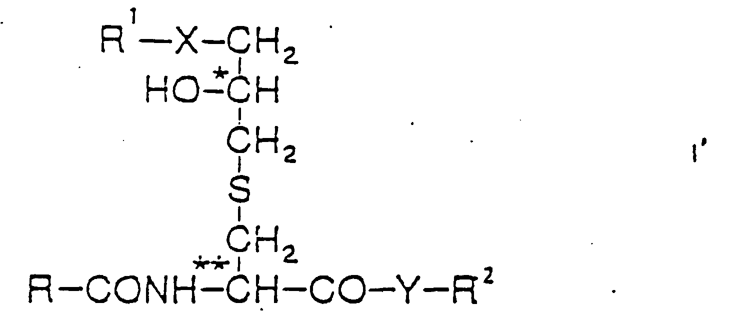 dans laquelle R représente le radical fluorénylméthyloxy, tert-butyloxy, benzyloxy ou un radical hydrocarboné saturé ou insaturé, aliphatique ou mixte aliphatique-cycloaliphatique, ayant de 1 à 21 atomes de carbone, éventuellement substitué par des atomes d'halogène ou des fonctions oxygénées, X représente un atome d'oxygène (O) ou le groupe méthylène (CH₂), Y représente un atome d'oxygène ou un reste d'aminoacide éventuellement protégé sur la chaîne latérale par un des groupes protecteurs usuels dans la chimie des peptides, R¹ représente un atome d'hydrogène, un radical hydrocarboné saturé ou insaturé, aliphatique ou mixte aliphatique-cycloaliphatique, ayant de 1 à 21 atomes de carbone, éventuellement substitué par des atomes d'halogène ou des fonctions oxygénées, ou un radical de formule C(O)-R³, R³ étant un radical hydrocarboné saturé ou insaturé, aliphatique ou mixte aliphatique-cycloaliphatique, ayant de 1 à 21 atomes de carbone, éventuellement substitué par des atomes d'halogène ou des fonctions oxygénées, et R² représente le groupe tert-butyle, méthyle, éthyle, propyle ou benzyle, à l'exclusion des composés dans lesquels R est un radical hydrocarboné, R¹ = H, X = O, Y = O et R² est le groupe tert-butyle, caractérisé en ce que l'on réduit un composé de formule II 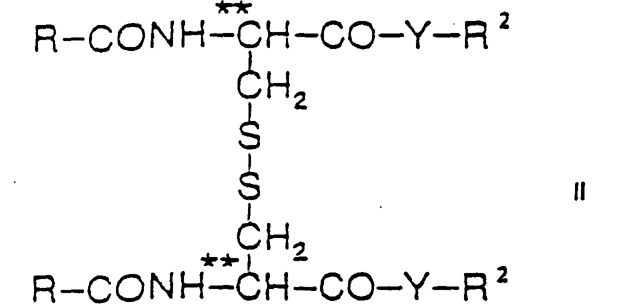 avec Zn dans un mélange solvant HCl/H₂SO₄ et on le fait réagir avec un composé de formule III 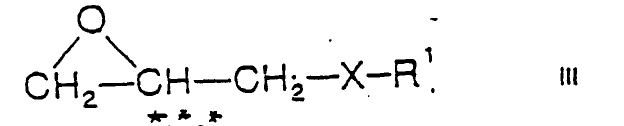 R, R¹, R², X et Y ayant les significations données plus haut.

3. Procédé pour la préparation des composés de formule I' selon la revendication 2, caractérisé en ce que R représente le radical fluorénylméthyloxy ou tert-butyloxy, ou un radical hydrocarboné aliphatique saturé ayant de 5 à 17 atomes de carbone, X représente un atome d'oxygène ou le groupe méthylène (CH₂) et Y représente un atome d'oxygène, R¹ représente un atome d'hydrogène, un radical hydrocarboné aliphatique saturé ayant de 7 à 18 atomes de carbone ou un radical de formule C(O)-R³, R³ étant un radical hydrocarboné saturé ayant de 5 à 17 atomes de carbone, et R² représente le groupe tert-butyle, de préférence à configuration absolue R au niveau des centres d'asymétrie désignés par * ou **.

4. Procédé pour la préparation des composés de formule I' selon la revendication 2 ou 3, caractérisé en ce que R représente le radical fluorénylméthyloxy ou un radical hydrocarboné aliphatique saturé ayant de 11 à 17 atomes de carbone, X représente un atome d'oxygène et Y représente un atome d'oxygène, R¹ représente un atome d'hydrogène, un radical hydrocarboné aliphatique saturé ayant de 9 à 18 atomes de carbone ou un radical de formule C(O)-R³, R³ étant un radical hydrocarboné saturé ayant de 9 à 17 atomes de carbone, et R² représente le groupe tert-butyle, de préférence à configuration absolue R au niveau des centres d'asymétrie désignés par * ou **.

5. Procédé selon les revendications 2, 3 ou 4, caractérisé en ce qu'il est effectué en tant que réaction en un seul récipient.

6. Utilisation des composés de formule I selon la revendication 1, pour la préparation de lipopeptides et de lipoaminoacides.

7. Utilisation des composés de formule I selon la revendication 1, pour la préparation de peptides contenant Pam₃CysOH et Pam₃Cys.

8. Utilisation des composés de formule I selon la revendication 1, pour la synthèse de lipopeptides dans la synthèse en phase solide, après élimination des groupes protecteurs de fonction acide correspondants.

9. Utilisation des composés de formule I selon la revendication 1 dans la synthèse en phase solide selon la revendication 8, caractérisée en ce que des groupes hydroxyle libres du peptide fixé à la résine sont estérifiés par des chlorures d'acides de formule IV 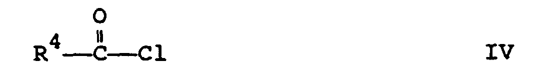 dans laquelle R⁴ représente un radical hydrocarboné ayant de 1 à 21 atomes de carbone.

## Revendications (Revendications pour l'(les) Etat(s) contractant(s) suivant(s): ES, GR)

1. Procédé pour la préparation des composés de formule I 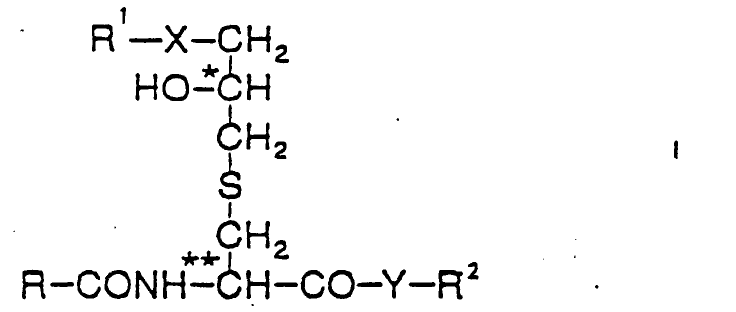 dans laquelle R représente le radical fluorénylméthyloxy, tert-butyloxy, benzyloxy ou un radical hydrocarboné saturé ou insaturé, aliphatique ou mixte aliphatique-cycloaliphatique, ayant de 1 à 21 atomes de carbone, éventuellement substitué par des atomes d'halogène ou des fonctions oxygénées, X représente un atome d'oxygène (O) ou le groupe méthylène (CH₂), Y représente un atome d'oxygène ou un reste d'aminoacide éventuellement protégé sur la chaîne latérale par un des groupes protecteurs usuels dans la chimie des peptides, R¹ représente un atome d'hydrogène, un radical hydrocarboné saturé ou insaturé, aliphatique ou mixte aliphatique-cycloaliphatique, ayant de 1 à 21 atomes de carbone, éventuellement substitué par des atomes d'halogène ou des fonctions oxygénées, ou un radical de formule C(O)-R³, R³ étant un radical hydrocarboné saturé ou insaturé, aliphatique ou mixte aliphatique-cycloaliphatique, ayant de 1 à 21 atomes de carbone, éventuellement substitué par des atomes d'halogène ou des fonctions oxygénées, et R² représente le groupe tert-butyle, méthyle, éthyle, propyle ou benzyle, à l'exclusion des composés dans lesquels R est un radical hydrocarboné, R¹ = H, X = O, Y = O et R² est le groupe tert-butyle, caractérisé en ce que l'on réduit un composé de formule II 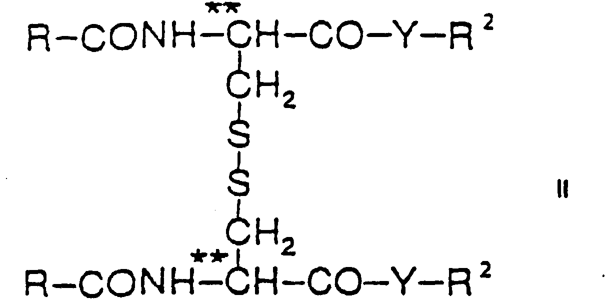 avec Zn dans un mélange solvant HCl/H₂SO₄ et on le fait réagir avec un composé de formule III 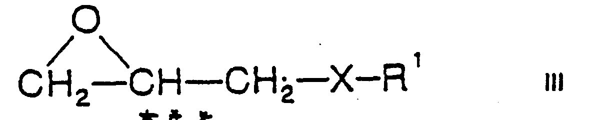 R, R¹, R², X et Y ayant les significations données plus haut.

2. Procédé pour la préparation des composés de formule I selon la revendication 1, caractérisé en ce que R représente le radical fluorénylméthyloxy ou tert-butyloxy, ou un radical hydrocarboné aliphatique saturé ayant de 5 à 17 atomes de carbone, X représente un atome d'oxygène ou le groupe méthylène (CH₂) et Y représente un atome d'oxygène, R¹ représente un atome d'hydrogène, un radical hydrocarboné aliphatique saturé ayant de 7 à 18 atomes de carbone ou un radical de formule C(O)-R³, R³ étant un radical hydrocarboné saturé ayant de 5 à 17 atomes de carbone, et R² représente le groupe tert-butyle, de préférence à configuration absolue R au niveau des centres d'asymétrie désignés par * ou **.

3. Procédé pour la préparation des composés de formule I selon la revendication 1 ou 2, caractérisé en ce que R représente le radical fluorénylméthyloxy ou un radical hydrocarboné aliphatique saturé ayant de 11 à 17 atomes de carbone, X représente un atome d'oxygène et Y représente un atome d'oxygène, R¹ représente un atome d'hydrogène, un radical hydrocarboné aliphatique saturé ayant de 9 à 18 atomes de carbone ou un radical de formule C(O)-R³, R³ étant un radical hydrocarboné saturé ayant de 9 à 17 atomes de carbone, et R² représente le groupe tert-butyle, de préférence à configuration absolue R au niveau des centres d'asymétrie désignés par * ou **.

4. Procédé pour la préparation des composés de formule I selon une ou plusieurs des revendications 1 à 3, caractérisé en ce que R représente le radical fluorénylméthyloxy, X représente un atome d'oxygène, Y représente un atome d'oxygène, R¹ représente un atome d'hydrogène, un radical hydrocarboné aliphatique saturé ayant de 9 à 18 atomes de carbone ou un radical de formule C(O)-R³, R³ étant un radical hydrocarboné saturé ayant de 9 à 17 atomes de carbone, et R² représente le groupe tert-butyle.

5. Procédé selon la revendication 1, caractérisé en ce qu'il est effectué en tant que réaction en un seul récipient.

6. Utilisation des composés de formule I selon la revendication 4, pour la préparation de lipopeptides et de lipoaminoacides.

7. Utilisation des composés de formule I selon la revendication 4, pour la préparation de peptides contenant Pam₃CysOH et Pam₃Cys.

8. Utilisation des composés de formule I selon la revendication 4, pour la synthèse de lipopeptides dans la synthèse en phase solide, après élimination des groupes protecteurs de fonction acide correspondants.

9. Utilisation des composés de formule I selon la revendication 4 dans la synthèse en phase solide selon la revendication 8, caractérisée en ce que des groupes hydroxyle libres du peptide fixé à la résine sont estérifiés par des chlorures d'acides de formule IV 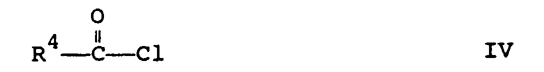 dans laquelle R⁴ représente un radical hydrocarboné ayant de 1 à 21 atomes de carbone.
